# EUROPEAN PATENT SPECIFICATION

(11) **EP 1 292 237 B1**
(45) Date of publication and mention of the grant of the patent: **19.11.2008**
(21) Application number: 01939441.0
(22) Date of filing: 25.05.2001
(51) Int. Cl.: A61B 17/34

(54) **TROCAR ASSEMBLY WITH CUSHIONED ACTIVATOR**
TROKARANORDNUNG MIT ELASTISCHEM AKTIVATOR
ENSEMBLE TROCART A ELEMENT D'ACTIONNEMENT REMBOURRE

(30) Priority: 25.05.2000 US 207082 P
(43) Date of publication of application: 19.03.2003
(73) Proprietor: Tyco Healthcare Group LP, Norwalk, Connecticut 06856 (US)
(72) Inventor: WELLS, Timothy, N., Ridgefield, CT 06677 (US); RACENET, David, C., Litchfield, CT 06759 (US)
(74) Representative: HOFFMANN EITLE
(86) International application number: PCT/US2001/016971
(87) International publication number: WO 2001/089399

(56) References cited:
- US-A- 5 312 363
- US-A- 5 389 077
- US-A- 5 674 237
- US-A- 5 853 392
- US-A- 5 928 154

## Description

### BACKGROUND

### 1. Technical Field

The present disclosure relates generally to a re-usable surgical instrument for puncturing a body cavity. More particularly, the present disclosure relates to a trocar assembly for puncturing a body cavity having a hand grip including a cushioned slip-resistant portion. The features of the pre-characterizing part of claim 1 below are disclosed in US-A-5389077.

### 2. Background of Related Art

Surgical instrumentation for puncturing body cavities, i.e., trocar assemblies are well known in the are Typically, a trocar assembly includes an obturator having a sharpened tip at one end for piercing the body cavity and a hand grip portion mounted on the other end of the obturator which the surgeon grasps in the palm of his hand. The hand grip portion includes a plunger which engages the other end of the obturator and can be pressed with the palm of the hand to force the sharpened end of the obturator through the body cavity wall Often, during endoscopic surgical procedures, multiple punctures through the body cavities are required.

In known trocar assemblies, the hand grip portion of the trocar assembly is formed from a hard plastic material and considerable force may be required to thrust the obturator through the body cavity wall. This force typically ranges from about 900 g (2 lbs.) to about 9 kg (20 lbs.) and may be even higher, especially when operating on obese individuals. Such a force may cause discomfort to and eventually bruising of the surgeon's hand. Moreover, during most surgical procedures, blood and other body fluids collect on a surgeon's hands or gloves making it difficult for the surgeon to grip the hand grip portion of the trocar assembly.

Further disclosures of background interest can be found in US-A-5312363, US-A-5928154 and US-A-5674237.

Accordingly, a need exists for an improved trocar assembly which can be actuated by a surgeon without causing the surgeon discomfort and which can be securely gripped by a surgeon even in the presence of body fluids.

### SUMMARY

In accordance with the present invention, there is provided a re-usable trocar assembly according to claim 1 which includes an obturator having a sharpened tip at one end and a hand grip secured to the other end. The cushioning member is removable, such that it can be removed from the surgical instrument prior to the sterilization and/or cleaning process. For example, the cushioning member could be provided as a removable flexible sleeve. The hand grip includes a cushioned, advantageously slip- resistant, member. The cushioned member is preferably formed from a thermoplastic elastomer, e.g., Versaflex^{™} or Santaprene^{™}, and over-molded onto the hand grip of the trocar assembly. Alternately, the cushioned member may be formed of other cushioned or pliant materials, e.g., elastomeric or synthetic materials, including isoprenes or nitrile or silicon-containing material, etc. Moreover, the grip member can be removably fastened to the grip portion using other known fastening techniques, e.g., physical, chemical or mechanical, including adhesives, screws, etc.

### BRIEF DESCRIPTION OF THE DRAWINGS

Various preferred embodiments of the presently disclosed trocar assembly are described herein with reference to the drawings, wherein:
FIG. 1 is a side cross-sectional view of the presently disclosed trocar assembly; and
FIG. 2 is a perspective view of one preferred embodiment of the presently disclosed trocar assembly positioned within a valved cannula assembly.

### DETAILED DESCRIPTION OF PREFERRED EMBODIMENTS

Preferred embodiments of the presently disclosed trocar assembly will now be described in detail with reference to the drawings in which like reference numerals designate identical or corresponding elements in each of the several views.

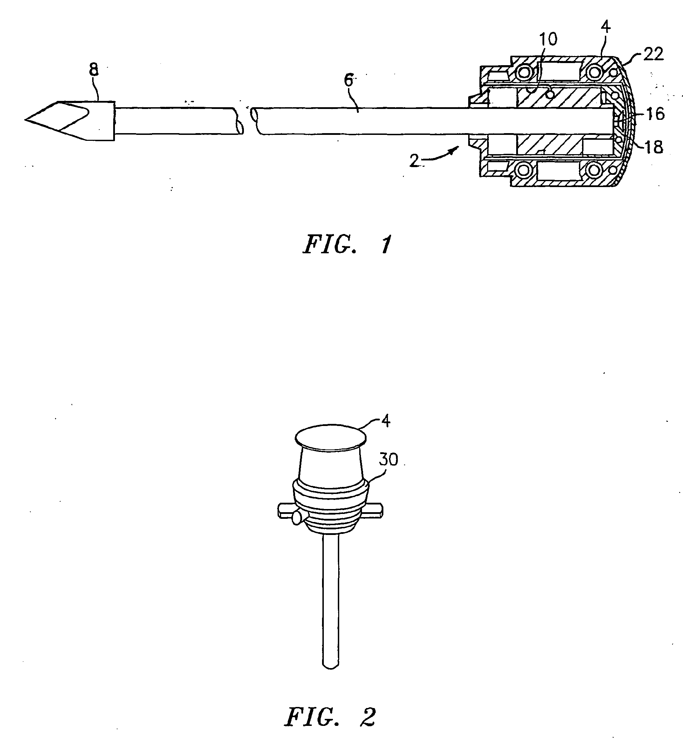

FIG. 1 illustrates a trocar assembly including an obturator 6 defining a longitudinal axis and having first and second ends. FIG. 2 illustrates the trocar assembly in combination with a cannula assembly 30. A sharpened tip 8 is mounted on the first end of the obturator 6. Tip 8 functions to penetrate or pierce a body cavity. A hand grip 4 is mounted on the second end of the obturator. Hand grip 4 is preferably formed from molded thermoplastic housing half-sections which are secured together to define a cavity 10 for receiving the second end of obturator 6. Alternately, other materials may be suitable for use, including other plastics, composites, surgical grade metals, etc. The pressure contact regions of the hand grip include those areas of hand grip 4 to which a surgeon must grasp or apply pressure to during manipulation of the trocar assembly or insertion of obturator 6 through tissue into a body cavity. In a preferred embodiment, cushioned grip member 22 is formed from a thermoplastic elastomer or elastomer blend, such a Versaflex^{™} or Santoprene^{™}, and is over-molded onto hand grip 4. A preferred thermoplastic elastomer is OM1040-X Versaflex^{™}. Alternately, the use of different cushioned or pliant materials is envisioned, as is the use of different techniques for fastening grip member 22 onto hand grip 4. For example, grip member 22 may be formed from other pliant materials, including plastics, elastomers, synthetics, etc. Moreover, grip member 22 may be fastened to hand grip 4 using other fastening techniques, e.g., chemical, physical, or mechanical, including adhesives, screws, welding, interengaging members, bonding, fusing, coating, dipping, spraying, etc.

The use of a cushioned portion formed from a thermoplastic or an elastomeric material on the pressure contact regions of the handle assembly cushions the impact on a surgeon's hand during operation of the surgical instrument Preferably, the cushioned portion is formed from a material having slip resistant properties which adhere well to the gloves worn by a surgeon, even in the presence of bodily fluids, to improve a surgeon's grip on the surgical instrument In addition, the cushioned material may include a textured, roughened or ridged surface to enhance or provide the slip-resistant surface.

The pressure required to actuate a particular instrument should be considered when choosing the material for forming the cushioned portion of the instrument. For example, a softer material may be more suitable for use with instruments requiring higher actuation pressures. Conversely, a harder material may be suitable for use in surgical instruments requiring smaller actuation pressures. The durometer of the cushioning material can be from about 10 to about 80, but is preferably between about 20 to about 50, and more preferably about 40.

## Claims

1. A re-usable trocar assembly comprising:
an obturator (6) defining a longitudinal axis and having first and second ends, a sharpened tip (8) positioned on the first end of the obturator and a hand grip (4) positioned on a second end of the obturator opposite the first end, and
**characterized by** a cushioned member (22) adapted to be positioned on at least one pressure contact surface of the hand grip (4), and adapted to be removed from the pressure contact surface prior to cleaning or sterilization of the trocar assembly.

2. A trocar assembly according to claim 1, wherein the cushioned member (22) is formed from an elastomeric material.

3. A surgical instrument according to claim 2, wherein the cushioned member (22) is over-molded onto the hand grip.

4. A trocar assembly according to claim 1, wherein the cushioned member (22) is in the form of a flexible sleeve.

5. A trocar assembly according to any one of the preceding claims, wherein the cushioned member (22) is formed from a slip resistant material.

6. A trocar assembly according to claim 2, wherein the elastomeric material is isoprene, nitrile or silicone.

## Patentansprüche

1. Wiederverwendbare Trokaranordnung, umfassend:
einen Obturator (6), der eine Längsachse definiert und ein erstes und zweites Ende, eine am ersten Ende des Obturators positionierte geschärfte Spitze (8) und einen dem ersten Ende gegenüber am zweiten Ende des Obturators positionierten Handgriff (4) aufweist, und **gekennzeichnet durch** ein gepuffertes Element (22), das geeignet ist, um auf mindestens einer Druckkontaktoberfläche des Handgriffs (4) positioniert zu werden, und geeignet ist, um vor der Reinigung oder Sterilisierung der Trokaranordnung von der Druckkontaktfläche entfernt zu werden.

2. Trokaranordnung nach Anspruch 1, wobei das gepufferte Element (22) aus einem elastomerischen Material gebildet ist.

3. Chirurgisches Instrument nach Anspruch 2, wobei der Handgriff mit dem gepufferten Element (22) umspritzt ist.

4. Trokaranordnung nach Anspruch 1, wobei das gepufferte Element (22) die Form einer flexiblen Hülse besitzt.

5. Trokaranordnung nach einem der vorangehenden Ansprüche, wobei das gepufferte Element (22) aus einem rutschfesten Material gebildet ist.

6. Trokaranordnung nach Anspruch 2, wobei das elastomerische Material Isopren, Nitril oder Silikon ist.

## Revendications

1. Ensemble trocart réutilisable comprenant:
un obturateur (6) définissant un axe longitudinal et ayant des première et seconde extrémités, une pointe affûtée (8) positionnée sur la première extrémité de l'obturateur et une poignée (4) positionnée sur une seconde extrémité de l'obturateur opposée à la première extrémité, et **caractérisé par** un élément rembourré (22) apte à être positionné sur au moins une surface de contact sous pression de la poignée (4) et apte à être retiré de la surface de contact sous pression avant le nettoyage ou la stérilisation de l'ensemble trocart.

2. Ensemble trocart selon la revendication 1, où l'élément rembourré (22) est réalisé à partir d'un matériau élastomère.

3. Instrument chirurgical selon la revendication 2, où l'élément rembourré (22) est surmoulé sur la poignée.

4. Ensemble trocart selon la revendication 1, où l'élément rembourré (22) se présente sous la forme d'un manchon flexible.

5. Ensemble trocart selon l'une quelconque des revendications précédentes, où l'élément rembourré (22) est réalisé à partir d'un matériau résistant au glissement.

6. Ensemble trocart selon la revendication 2, où le matériau élastomère est de l'isoprène, nitrile ou silicone.
